(19) Europäisches Patentamt
European Patent Office
Office européen des brevets

(11) Publication number: 0 150 463 A1

# (12) EUROPEAN PATENT APPLICATION

(21) Application number: 84116018.7

(22) Date of filing: 20.12.84

(51) Int. Cl.4: C 07 D 403/10, C 07 D 409/14

(30) Priority: 20.01.84 DE 3401911

(43) Date of publication of application: 07.08.85 Bulletin 85/32

(84) Designated Contracting States: AT BE CH DE FR GB IT LI LU NL SE

(71) Applicant: A. Nattermann & Cie. GmbH, Nattermannallee 1, D-5000 Köln 30 (DE)

(72) Inventor: Hilboll, Gerd, Dr., Dehmelstrasse 36, D-5000 Köln 30 (DE)
Inventor: Prop, Gerrit, Dr., Anemonenweg 23, D-5024 Pulheim (DE)
Inventor: Borbe, Harald O., Dr., Frechener Weg 30, D-5000 Köln 40 (DE)
Inventor: Uhlendorf, Joachim, Dr., Am Beissel 14, D-5042 Erftstadt 12 (DE)

(74) Representative: Redies, Bernd, Dr. rer. nat. et al, Redies, Redies, Türk & Gille, Patentanwälte Brucknerstrasse 20, D-4000 Düsseldorf 13 (DE)

## (54) Substituted 4.5-dihydro-6-vinyl-3(2H)-pyridazinones and 6-vinyl-3(2H)-pyridazinones and process for producing the same.

(57) The invention is related to new substituted 4.5-dihydro-6-vinyl-3(2H)-pyridazinones and 6-vinyl-3(2H)-pyridazinones of the formula I

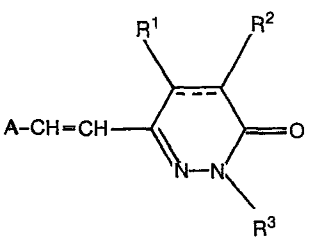

I

as well as to processes for producing the same.

EP 0 150 463 A1

0150463

SUBSTITUTED 4.5-DIHYDRO-6-VINYL-3(2H)-PYRIDAZINONES AND 6-VINYL-3(2H)-PYRIDAZINONES AND PROCESS FOR PRODUCING THE SAME

Specification

The invention is related to new 4.5-dihydro-6-vinyl-3(2H)-pyridazinones and 6-vinyl-3(2H)-pyridazinones, process for producing the same, pharmaceutical preparations containing these compounds and their use as drugs in the treatment of diseases.

4.5-Dihydro-6-/2-(pyridyl)-ethenyl/-3(2H)-pyridazinones and 6-/2-(Pyridyl)-ethenyl/-3(2H)-pyridazinones are already described in European patent application EP 81906 as having cardiotonic and/or antihypertensive properties.

It now has been found that substituted 4.5-dihydro-6-vinyl-3(2H)-pyridazinones and 6-vinyl-3(2H)-pyridazinones of formula I

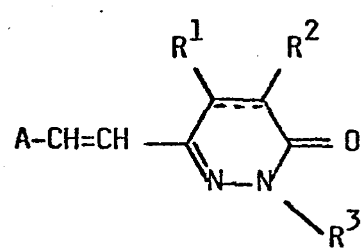

I

wherein

$R^1$, $R^2$ and $R^3$ independently represent hydrogen or a methyl group,

--- is a double bond or a single bond between two carbon atoms and

A is a member of the group of

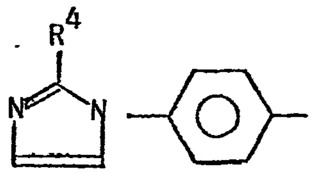 and 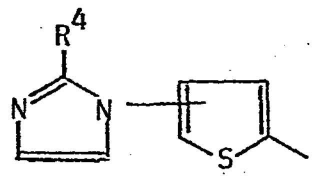

$R^4$ being a hydrogen or a methyl group

have valuable pharmacoligical properties in particular blood pressure lowering and antithrombotic properties. They are therfore useful in the treatment of high blood pressure and/or thrombosis and in the prevention of such conditions, in human beings.

Included are also the pharmacologically acceptable salts of the compounds of formula I with inorganic or organic acids such as hydrochlorides, acetates, fumarates, citrates, lactates and benzoates.

The compounds of formula I wherein $R^3$ is hydrogen, are in balance with the corresponding tautomeric 4.5-dihydro-3-hydroxy-pyridazines ( $\overline{...}$ being a single bond) or, respectively, 3-hydroxy-pyridazines ( $\overline{...}$ being a double bond).

The 4.5-dihydro-6-vinyl-3(2H)-pyridazinones of formula I have a chirality center in position 4 or, respectively, 5 of the pyridazine ring if the substituents $R^1$ and/or $R^2$ are different from hydrogen and, therefore, they may be present as racemates or as the corresponding enantiomers. If the separation of the racemates is desired, separation is effected by the processes known for this purpose, by means of an optically active acid such as dibenzoyl tataric acid or campher-10-sulfonic acid with the formation of diastereomeric salts or by column chromatography on optically active adsorbents. Alternatively, the enantiomers may be obtained by separate reaction of optically active starting materials.

Compounds according to the invention are for instance:

0150463

4,5-Dihydro-6-[2-[4-(1-imidazolyl)-phenyl]-ethenyl]-3(2H)-pyridazinone

4,5-Dihydro-6-[2-[4-(2-methyl-1-imidazolyl)-phenyl]-ethenyl]-3(2H)-pyridazinone

4,5-Dihydro-6-[2-[5-(1-imidazolyl)-thien-2-yl]-ethenyl]-3(2H)-pyridazinone

4,5-Dihydro-6-[2-[4-(1-imidazolyl)-thien-2-yl]-ethenyl]-3(2H)-pyridazinone

4,5-Dihydro-6-[2-[5-(2-methyl-1-imidazolyl)-thien-2-yl]-ethenyl]-3(2H)-pyridazinone

4,5-Dihydro-6-[2-[4-(1-imidazolyl)-phenyl]-ethenyl]-5-methyl-3(2H)-pyridazinone

4,5-Dihydro-5-methyl-6-[2-[4-(2-methyl-1-imidazolyl)-phenyl]-ethenyl]-3(2H)-pyridazinone

4,5-Dihydro-6-[2-[5-(1-imidazolyl)-thien-2-yl]-ethenyl]-5-methyl-3(2H)-pyridazinone

4,5-Dihydro-6-[2-[4-(1-imidazolyl)-thien-2-yl]-ethenyl]-5-methyl-3(2H)-pyridazinone

4,5-Dihydro-5-methyl-6-[2-[5-(2-methyl-1-imidazolyl)-thien-2-yl]-ethenyl]-3(2H)-pyridazinone

4,5-Dihydro-6-[2-[4-(1-imidazolyl)-phenyl]-ethenyl]-4-methyl-3(2H)-pyridazinone

4,5-Dihydro-4-methyl-6-[2-[4-(2-methyl-1-imidazolyl)-phenyl]-ethenyl]-3(2H)-pyridazinone

4,5-Dihydro-6-[2-[5-(1-imidazolyl)-thien-2-yl]-ethenyl]-4-methyl-3(2H)-pyridazinone

4,5-Dihydro-6-[2-[4-(1-imidazolyl)-thien-2-yl]ethenyl]-4-methyl-3(2H)-pyridazinone

4,5-Dihydro-6-[2-[4-(1-imidazolyl)-phenyl]-ethenyl]-2-methyl-3(2H)-pyridazinone

4,5-Dihydro-6-[2-[5-(1-imidazolyl)-thien-2-yl]-ethenyl]-2-methyl-3(2H)-pyridazinone

4,5-Dihydro-6-[2-[4-(1-imidazolyl)-thien-2-yl]-ethenyl]-2-methyl-3(2H)-pyridazinone

4,5-Dihydro-2,5-dimethyl-6-[2-[4-(1-imidazolyl)-phenyl]-ethenyl]-3(2H)-pyridazinone

6-[2-[4-(1-Imidazolyl)-phenyl]-ethenyl]-3(2H)-pyridazinon

6-[2-[4-(2-Methyl-1-imidazolyl)-phenyl]-ethenyl]-3(2H)-pyridazinone

6-[2-[5-(1-Imidazolyl)-thien-2-yl]-ethenyl]-3(2H)-pyridazinone

6-[2-[4-(1-Imidazolyl)-thien-2-yl]-ethenyl]-3(2H)-pyridazinone

6-[2-[5-(2-Methyl-1-imidazolyl)-thien-2-yl]-ethenyl]-3(2H)-pyridazinone

6-[2-[4-(1-Imidazolyl)-phenyl]-ethenyl]-5-methyl-3(2H)-pyridazinone

5-Methyl-6-[2-[4-(2-methyl-1-imidazolyl)-phenyl]-ethenyl]-3(2H)-pyridazinone

6-[2-[5-(1-Imidazolyl)-thien-2-yl]-ethenyl]-5-methyl-3(2H)-pyridazinone

6-[2-[4-(1-Imidazolyl)-thien-2-yl]-ethenyl]-5-methyl-3(2H)-pyridazinone

5-Methyl-6-[2-[5-(2-methyl-1-imidazolyl)-thien-2-yl]-ethenyl]-3(2H)-pyridazinone

6-[2-[4-(1-Imidazolyl)-phenyl]-ethenyl]-4-methyl-3(2H)-pyridazinone

4-Methyl-6-[2-[4-(2-methyl-1-imidazolyl)-phenyl]-ethenyl]-3(2H)-pyridazinone

6-[2-[5-(1-Imidazolyl)-thien-2-yl]-ethenyl]-4-methyl-3(2H)-pyridazinone

6-[2-[4-(1-Imidazolyl)-thien-2-yl]-ethenyl]-4-methyl-3(2H)-pyridazinone

6-[2-[4-(1-Imidazolyl)-phenyl]-ethenyl]-2-methyl-3(2H)-pyridazinone

6-[2-[5-(1-Imidazolyl)-thien-2-yl]-ethenyl]-2-methyl-3(2H)-pyridazinone

6-[2-[4-(1-Imidazolyl)-thien-2-yl]-ethenyl]-2-methyl-3(2H)-pyridazinone

2,5-Dimethyl-6-[2-[4-(1-imidazolyl)-phenyl]-ethenyl]-3(2H)-pyridazinone

The production of the compounds according to the invention is effected according to known processes (EP 81906). The 4.5-dihydro-6-vinyl-3(2H)-pyridazinones of formula I ( --- being single bond) are produced by reaction of the 4-oxo-5-hexenic acids of formula II or their alkyl esters, wherein A, $R^1$, $R^2$ have the meaning as in formula I, with a hydrazine derivative of formula III, wherein A has the meaning as in formula I, or a hydrate or salt thereof such as a hydrochloride, hydrogenosulfate, sulfate or the like, in an aqueous, aqueous alcoholic, alcoholic medium or in solvents inert under the selected reaction conditions, such as dioxane, toluene, dimethylformamide or mixtures thereof with water and/or alcohol, at temperatures ranging from 30 to 150°C, preferably from 30 to 100°C in water or alcohol, possibly with the aid of one of the catalysts usual for aminolysis and condensation reactions.

The reactions follow the following formulas:

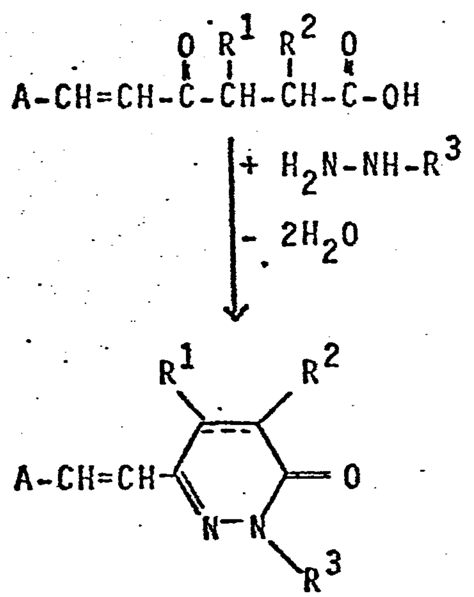


II

III

I

(--- single bond)

Hydrazine derivatives of formula III are in particular:

Hydrazine or methylhydrazine and their hydrates or salts, such as hydrochlorides, hydrogenosulfates, sulfates.

The preparation of the 4-oxo-5-hexanic acids of formula II used as starting materials is effected by manners known per se (EP 81906)

in that an aldehyde of formula V

$$A-\overset{O}{\overset{\|}{C}}-H \qquad \text{V}$$

wherein A has the meaning as given in formula I, is reacted with a 4-oxo-pentanoic acid of formula VI

$$CH_3-\overset{C}{\overset{\|}{C}}-\overset{R^1}{\overset{|}{C}H}-\overset{R^2}{\overset{|}{C}H}-\overset{O}{\overset{\|}{C}}-OH \qquad \text{VI}$$

wherein $R^1$ and $R^2$ have the same meaning as in formula I, under conditions which are typical for a condensation reaction such as by heating equimolar amounts in an aromatic hydrocarbon such as benzene, toluene, in the presence of catalytic amounts of a base, such as piperidine, with simultaneous continues removal of the water resulting in this reaction.

The 6-vinyl-3(2H)-pyridazinones of formula I ( ⋯ being a double bond) are produced according to know processes (EP 81906) from the above cited 4.5-dihydro-6-vinyl-3(2H)-pyridazinones of formula I ( ⋯ being a single bond) by reacting them with dehydrogenation agents such as bromium, selenium dioxide, preferably by reaction with 3-nitrobenzene sulfonic acids in an alkaline aqueous reaction medium at temperatures ranging from 60 to 100°C. The reaction follows the following formula:

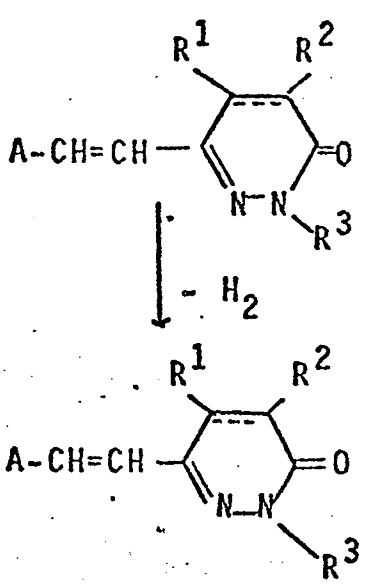


I
( ⋯ single bond)

I
( ⋯ single bond)

The acid addition salts of compounds of formula I with inorganic or organic acids may be produced by mixing the basic compounds with the corresponding acids in aqueous, aqueous organic, for instance aqueous alcohol, or organic reaction medium such as alcohols, alcohol -ether-mixtures or ether-petrolether-mixtures, at temperatures between 0 and 100°C.

The present invention is further related to pharmaceutical preparations containing compounds of formula I or pharmaceutically acceptable acid addition salts thereof. The pharmaceutical preparations according to the invention are preparations for enteral or oral or rectal as well as parenteral administration which contain the pharmaceutically active agents alone or together with a usual pharmaceutical carrier material. Preferably, the pharmaceutical preparation of the active agent are present in single dosage form in accordance with the intended administration, such as tablets, dragees, capsules, suppositories, granulates, solvents, emulsions or suspensions. The dosages of the compounds is generally between 1 to 500 mg per dose, preferably between 10 to 150 mg per dose and may be administered one or several times per day, preferably two or three times per day.

The production of the compounds according to the present invention is further illustrated by the following examples. Melting points there cited have been determined in a Büchi 510 melting point determination apparatus. Melting points are given in °C and are not corrected. Infrared spectra have been determined in an apparatus Perkin Elmer 257 or Nicolet NIC-3600 and mass spectra have been determined in an apparatus Varian MAT-311A (70eV).

0150463

Example 1

4.5-Dihydro-6-[2-[4-(1-imidazolyl)-phenyl]-ethenyl]-3(2H)-pyridazinone.

a) 6-[4-(1-Imidazolyl)-phenyl]-4-oxo-5-hexenoic acid

30 g of 4-(1-imidazolyl)-benzaldehyde and 20.2 g of 4-oxo-pentanoic acid with 7 ml of piperidine and 350 ml of toluene are refluxed with continuous separation of the water produced during the reaction. After the separation of water has come to an end, the solid product precipitated during the reaction is filtered off after cooling, washed with toluene and hexane and is dried.

Yield: 42.5 g     Point of Decomposition: 228 to 230°C

IR (in KBr): 1710, 1680, 1603 $cm^{-1}$

MS [m/e]: 270 ($M^+$, 38%), 197 (100%), 169 (12%), 115 (13%)

b) 4.5-Dihydro-6-[2-[4-(1-imidazolyl)-phenyl]-ethenyl]-3(2H)-pyridazinone

30 g of6-[4-(1-imidazolyl)-phenyl]-4-oxo-5-hexenoic acid (Example 1a) are heated to boiling in 350 ml of methanol. 7.2 g of hydrazine -hydrate is added to the hot suspension. Thereafter, the reaction mixture is stirred for 4 hours allowing a slow cooling to room temperature. The solid precipitated material is filtered off with suction and is recrystallized from ethanol.

Yield: 8.7 g     M.p.: 231 to 233°C

IR (in KBr): 1662, 1608 $cm^{-1}$

MS [m/e]: 266 ($M^+$, 100%), 265 (98%), 237 (5%),
222 (5%), 194 (5%), 168 (4%), 141 (5%),
128 (9%), 115 (6%)

0150463

Example 2

4.5-Dihydro-6-[2-[4-(2-methyl-1-imidazolyl)-phenyl]-ethenyl]-3(2H)-pyridazinone.

a) 6-[4-(2-Methyl-1-imidazolyl)-phenyl]-4-oxo-5-hexenoic acid.

The reaction is carried out as in Example 1a) with 18.6 g of 4-(2-methyl-1-imidazolyl)-benzaldehyde, 11.6 g of 4-oxo-pentanoic acid, 4 ml of piperidine and 200 ml of toluene.

Yield: 7.6 g M.p.: 212°C

IR (in KBr): 1715, 1680, 1605 $cm^{-1}$

MS [m/e] : 284 ($M^+$, 61 %), 211 (100 %), 183 (5 %), 142 (11 %), 115 (15 %), 102 (17 %)

b) 4.5-Dihydro-6-[2-[4-(2-methyl-1-imidazolyl)-phenyl]-ethenyl]-3(2H)-pyridazinone.

The reaction is carried out as in Example 1b) with 3.8 g of 6-[4-(2-methyl-1-imidazolyl)-phenyl]-4-oxo-5-hexenoic acid (Example 2a) and 0.8 ml of hydrazine-hydrate in 50 ml of methanol.

Yield: 1.3 g M.p.: 201 to 203°C (from ethanol)

IR (in KBr): 1665, 1604 $cm^{-1}$

MS [m/e] : 280 $M^+$, 100 %), 279 (97 %), 236 (5 %), 167 (5 %), 141 (8 %), 128 (11 %), 115 (5 %)

Example 3

4.5-Dihydro-6-[2-[4-(1-imidazolyl)-thien-2-yl]-ethenyl]-3(2H)-pyridazinone.

a) 6-[4-(1-Imidazolyl)-thien-2-yl]-4-oxo-5-hexenoic acid.

The reaction is carried out as in Example 1a) with 17.8 g of 4-(1-imidazolyl)-thiophen-2-aldehyde (produced from imidazol and 4-bromothiophen-2-aldehyde, M.p. 127 to 130°C), 11.6 g of 4-oxo-pentanoic acid, 2.5 ml of piperidine and 200 ml of toluene. After the reaction has been carried out, the reaction mixture is evaporated, the residue is triturated with ethanol, the solid material is filtered off and dried.

Yield: 9.7 g M.p.: 245°C

IR (in KBr): 3084, 1685, 1604 $cm^{-1}$

MS [m/e] : 276 ($M^+$, 44 %), 249 (7 %), 203 (100 %), 175 (8 %).

b) 4.5-Dihydro-6-[2-[4-(1-imidazolyl)-thien-2-yl]-ethenyl]-3(2H)-pyridazinone.

The reaction is carried out as in Example 1b) with 5.5 g of 6-[4-(1-Imidazolyl)-thien-2-yl]-4-oxo-5-hexenoic acid (Example 10a), 1.2 ml of hydrazine-hydrate and 100 ml of methanol.

Yield: 2.4 g M.p.: 275 to 277°C

IR (in KBr): 1655, 1621 $cm^{-1}$

MS [m/e] : 272 ($M^+$, 100 %), 243 (6 %), 228 (11 %), 180 (14 %)

0150463

Example 4

4.5-Dihydro-6-[2-[4-(1-imidazolyl)-phenyl] -ethenyl]-2-methyl-3(2H)-pyridazinone.

The reaction is carried out as described in Example 1b) with 4.3 g of 6-[4-(1-imidazolyl)-phenyl]-4-oxo-5-hexenoic acid (Example 1a), 1 ml of methylhydrazine and 30 ml of methanol. The crude reaction product is filtered off with suction and is recrystallized from ethanol.

Yield: 0.5 g M.p.: 205°C

IR (in KBr): 1643, 1605 $cm^{-1}$

MS [m/e] : 280 ($M^+$, 100 %), 251 (7 %), 237 (8 %),
209 (7 %), 195 (10 %)

Example 5

4.5-Dihydro-6-[2-[4-(1-imidazolyl)-thien-2-yl]-ethenyl]-2-methyl-3(2H)-pyridazinone.

The reaction is carried out as described in Example 1b) with 7.2 g of 6-[4-(1-imidazolyl)-thien-2-yl]-4-oxo-5-hexenoic acid (Example 3a), 0.64 ml of hydrazine-hydrate and 20 ml of methanol. The crude reaction product is purified by column chromatography ($SiO_2$-gel// chloroform/methanol). Thereafter, it is recrystallized from ethanol.

Yield: 0.15 g M.p.: 182°C

IR (in KBr): 1649 $cm^{-1}$

MS [m/e] : 286 ($M^+$, 100 %), 257 (7 %), 243 (7 %),
215 (6 %), 202 (8 %), 201 (9 %), 174 (5 %),
147 (7 %), 137 (8 %), 57 (29 %)

Example 6

6-[2-[4-(1-Imidazolyl)-phenyl]-ethenyl]-3(2H)-pyridazinone.

A mixture of 4 g of 4.5-dihydro-6-[2-[4-(1-imidazolyl)-phenyl]-ethenyl]-3(2H)-pyridazinone (Example 1b), 3.7 g of the sodium salt of 3-nitrobenzene sulfonic acid, 2.6 g of NaOH and 38 ml of water is stirred and refluxed for 2 hours. Thereafter, a small amount of activated carbon is added and heated is continued for another 5 minutes. The resulting reaction mixture is filtrated hot and is neutralized by the addition of acetic acid. After cooling, the precipitated solid material is filtered off and is purified by column chromatography ($SiO_2$-gel//chloroform/methanol).

Yield: 0.2 g M.p.: 238 to 240°C

IR (in KBr): 1674, 1656, 1607 $cm^{-1}$

MS [m/e] : 264 ($M^+$, 100 %), 263 (82 %), 139 (16 %)

Patent claims

1. Substituted 4.5-dihydro-6-vinyl-3(2H)-pyridazinones and 6-vinyl-3(2H)-pyridazinones of formula I

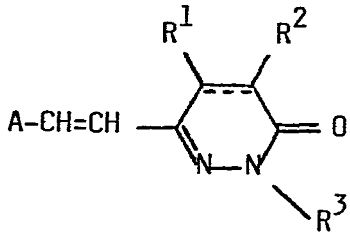

I

wherein

$R^1$, $R^2$ and $R^3$ independently represent hydrogen or a methyl group,

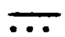 is a double bond or a single bond between two carbon atoms,

A is a member of the group of

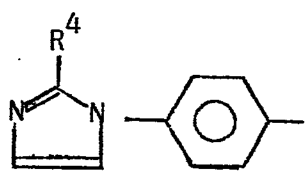 and 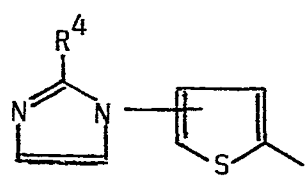

and

$R^4$ is hydrogen or a methyl group.

2. A compound as claimed in claim 1 wherein in formula I $R^1$, $R^2$, $R^3$ and  have the same meaning as in claim 1 and A is

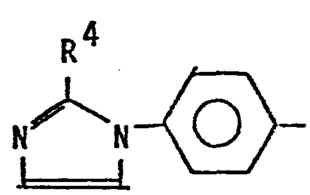

$R_4$ being hydrogen or a methyl group.

3. A compound as claimed in claim 1 wherein in formula I, $R^1$, $R^2$, $R^3$ and $\overline{\cdots}$ have the same meaning as in claim 1 and A is

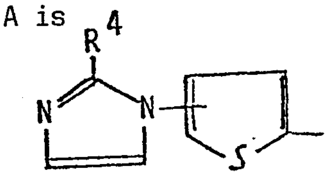

$R^4$ being hydrogen or a methyl group.

4. Process for the production of compounds of formula I, claims 1 to 3 ( $\overline{\cdots}$ being a simple bondage), characterized in that an 4-oxo-5-hexene acid of formula II or its alkyl esters

$$A\text{-}CH{=}CH\text{-}\overset{\overset{O}{\|}}{C}\text{-}\overset{\overset{R^1}{|}}{C}H\text{-}\overset{\overset{R^2}{|}}{C}H\text{-}\overset{\overset{O}{\|}}{C}\text{-}OH \qquad II$$

wherein A, $R^1$, $R^2$ have the same meaning as in formula I, claim 1, is reacted with a hydrazine compound of formula III

$$H_2N\text{-}NH\text{-}R^3 \qquad III$$

wherein $R^3$ has the same meaning as in formula I, or a salt or hydrate thereof, in an aqueous, aqueous-alcoholic or alcoholic reaction medium or in another solvent inert under the selected reaction conditions such as dioxane or dimethylformamide or mixtures thereof with water and/or alcohol at temperatures of from 30 to 150°C, preferably of from 30 to 100°C in water or alcohol, possibly with the aid of a catalyst usual for aminolysis- and condensation reactions.

5. Process for the production of compounds of formula I, claims 1 to 3 ( $\overline{\cdots}$ being a double bond), characterized in that a 4.5-dihydro-6-vinyl-3(2H)-pyridazinone of formula I ( $\overline{\cdots}$ being a single bond) is reacted with a dehydration agent such as bromium, selenium dioxide, preferably the sodium salt of 3-nitrobenzene sulfonic acid, in an aqueous-alkaline reaction medium at temperatures ranging from 60 to 100°C.

European Patent Office

# EUROPEAN SEARCH REPORT

0150463
Application number

EP 84116018.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | DE - A1 - 3 130 251 (NATTERMANN)<br>* Formula I *<br>-- | 1 | C 07 D 403/10<br>C 07 D 409/14 |
| A | DE - A1 - 3 130 252 (NATTERMANN)<br>* Formula I *<br>-- | 1 | |
| A | EP - A1 - 0 075 436 (WARNER)<br>* Formula IV *<br>-- | 1 | |
| A | CHEMICAL ABSTRACTS, vol. 97, no. 21, November 22, 1982<br>Columbus, Ohio, USA<br>ISMAIL, M. FEKRY; SHAMS, NABIL A.; MOSTAFA, OMNIA E. "Synthesis of 6-(αstyryl)pyridazin-3(2H)-ones.<br>page 821, column 1, abstract no. 182 331s<br>& Indian J. Chem., Sect. B, 1982, 21B(4), 371-2<br>---- | 1 | TECHNICAL FIELDS SEARCHED (Int. Cl.4)<br>C 07 D 403/00<br>C 07 D 409/00 |

The present search report has been drawn up for all claims

| Place of search | Date of completion of the search | Examiner |
|---|---|---|
| VIENNA | 25-03-1985 | HAMMER |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document